# EUROPÄISCHE PATENTSCHRIFT

(11) **EP 1 035 879 B1**
(45) Veröffentlichungstag und Bekanntmachung des Hinweises auf die Patenterteilung: **11.06.2003**
(21) Anmeldenummer: 98966494.1
(22) Anmeldetag: 24.11.1998
(51) Int. Cl.: A61L 27/28, A61L 27/34, A61L 31/08, A61L 31/10

(54) **VERFAHREN ZUR IMMOBILISIERUNG VON MEDIATORMOLEKÜLEN AUF ANORGANISCHEN UND METALLISCHEN IMPLANTATMATERIALIEN**
METHOD FOR IMMOBILIZING MEDIATOR MOLECULE ON INORGANIC AND METAL IMPLANT MATERIAL
PROCEDE POUR IMMOBILISER DES MOLECULES MEDIATRICES SUR DES MATERIAUX D'IMPLANTS INORGANIQUES ET METALLIQUES

(30) Priorität: 24.11.1997 DE 19752032
(43) Veröffentlichungstag der Anmeldung: 20.09.2000
(73) Patentinhaber: EFMT Entwicklungs- und Forschungszentrum für Mikrotherapie gGmbH, 44799 Bochum (DE)
(72) Erfinder: Jennissen, Herbert P., Prof. Dr., 45279Essen (DE)
(74) Vertreter: Viering, Jentschura & Partner
(86) Internationale Anmeldenummer: DE9803463
(87) Internationale Veröffentlichungsnummer: WO99026674

(56) Entgegenhaltungen:
- WO-A-90/09798
- WO-A-92/00047
- GB-A- 387 806
- GB-A- 593 287
- US-A- 5 330 911

## Beschreibung

Die vorliegende Erfindung betrifft ein Verfahren zur Immobilisierung von Mediatormolekülen auf Oberflächen von metallischen oder keramischen Materialien, die für Implantate wie künstliche Gelenke oder auch Kleinstimplante, z.B. sogenannte Stents, verwendet werden, als auch nach dem Verfahren hergestellte Implantate.

Die Implantation künstlicher Gelenke oder Knochen hat in den letzen Jahren eine zunehmende Bedeutung z.B. bei der Behandlung von Gelenkdysplasien oder -luxationen bzw. bei Erkrankungen, die auf der Abnutzung von Gelenken als Folge von Gelenkfehlstellungen entstehen "können, gewonnen. Die Funktion der Implantate und die für ihre Herstellung verwendeten Materialien, die neben Metallen wie Titan oder Metallegierungen auch Keramik oder Kunststoffmaterialien wie Teflon® umfassen können, sind stetig verbessert worden, so dass Implantate nach erfolgreichem Heilungsverlauf in 90-95% der Fälle Standzeiten von 10 Jahren aufweisen können. Ungeachtet dieser Fortschritte und verbesserter operativer Verfahren bleibt eine Implantation immer noch ein schwieriger und belastender Eingriff, insbesondere da sie mit einem langwierigen Einheilungsprozeß des Implantates, der oft monatelange Klinik- und Kuraufenthalte einschließlich Rehabilitationsmaßnahmen umfaßt, verbunden ist. Neben den Schmerzen stellen dabei für die betroffenen Patienten die Länge der Behandlungsdauer und die Trennung von der vertrauten Umgebung große Belastungen dar. Ferner verursacht der langwierige Heilungsprozeß durch die erforderliche intensive Betreuung hohe Personal- und Pflegekosten.

Das Verständnis der Vorgänge auf der molekularen Ebene, die für ein erfolgreiche Einwachsen eines Implantates erforderlich sind, hat sich in den letzten Jahren bedeutend erweitert. Entscheidend für die Gewebeverträglichkeit eines Implantates sind Strukturkompatibilität und Oberflächenkompatibilität. Die Biokompatibilität im engeren Sinne ist alleine von der Oberfläche bedingt. Auf allen Ebenen der Integration spielen Proteine eine maßgebliche Rolle. Wie nachfolgend erläutert, entscheiden sie bereits während der Implantationsoperation durch die Ausbildung einer initialen adsorbierten Proteinschicht über den weiteren Verlauf der Implantateinheilung, da sich auf dieser Schicht später die ersten Zellen ansiedeln.

Bei der molekularen Interaktion zwischen Implantat, das auch als Biomaterial bezeichnet wird, und Gewebe, findet eine Vielzahl von Reaktionen statt, die streng hierarchisch geordnet zu sein scheinen. Als erste biologische Reaktion findet die Adsorption von Proteinen an der Oberfläche des Biomaterials statt. In der dadurch entstandenen Proteinschicht werden anschließend einzelne Proteinmoleküle beispielsweise entweder durch Konformationsänderungen zu Signalstoffen umgewandelt, die auf der Oberfläche präsentiert werden, oder durch katalytische (proteolytische) Reaktionen werden als Signalstoffe wirkende Proteinfragmente freigesetzt. Ausgelöst durch die Signalstoffe, findet in der nächsten Phase die zelluläre Besiedlung statt, die eine Vielzahl von Zellen wie Leukozyten, Makrophagen, Immunozyten, und schließlich auch Gewebezellen (Fibroblasten, Fibrozyten, Osteoblasten, Osteozyten) umfassen kann. In dieser Phase spielen andere Signalstoffe, sogenannte Mediatoren, wie z.B. Cytokine, Chemokine, Morphogene, Gewebshormone und echte Hormone eine entscheidende Rolle. Im Falle einer Biokompatibilität kommt es schließlich zur Integration des Implantates in den Gesamtorganismus, und idealerweise erhält man ein Permanentimplantat.

Im Licht von Arbeiten, die in den letzten Jahren auf molekularer Ebene der Osteogenese durchgeführt worden sind, haben chemische Signalstoffe, die sogenannten "bone morphogenic Proteins" (BMP-1-BMP-13), die Einfluß auf das Knochenwachstum besitzen, eine zunehmende Bedeutung gewonnen. BMPs (insbesondere BMP-2 und BMP-4, BMP-5, BMP-6, BMP-7) sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von Hormonrezeptoren, knochenspezifischer Substanzen wie Kollagen Typ 1, Osteocalcin, Osteopontin und schließlich die Mineralisation. Die BMP-Moleküle regulieren dabei die drei Schlüsselreaktionen Chemotaxis, Mitose und Differenzierung der jeweiligen Vorläuferzelle. Darüber hinaus spielen BMPs eine wichtige Rolle in der Embryogenese, Organogenese des Knochens und anderer Gewebe, wobei als Zielzellen Osteoblasten, Chondroblasten, Myoblasten und vaskulare glatte Muskelzellen (Proliferationshemmung durch BMP-2) bekannt ist.

Bei dem erfindungsgemäßen Immobilisierungsverfahren wird insbesondere ein Substitutionsgrad (d.h. Oberflächenkonzentration des immobilisierten Proteins) angestrebt, der eine multivalente Wechselwirkung zwischen Oberfläche und Zelle erlaubt und ermöglicht, die Knochen- oder Gewebsbildung wirksam zu steuern.

Inzwischen sind 13 BMPs inklusive multipler Isoformen bekannt. Bis auf das BMP-1 gehören die BMPs der "transforming growth factor beta" (TGF-β) Superfamilie an, für die spezifische Rezeptoren auf den Oberflächen der entsprechenden Zellen nachgewiesen wurden. Wie der erfolgreiche Einsatz von rekombinanten humanen BMP-2 und/oder BMP-7 in Versuchen zu Defektheilungsprozessen an Ratten, Hunden, Kaninchen und Affen gezeigt hat, scheint keine Speziesspezifität vorzuliegen. Bisherige Versuche, die knochenbildungsauslösenden Eigenschaften der BMPs gezielt für Implantationszwecke auszunutzen, indem das BMP-2 und/oder BMP-7 nicht-kovalent auf metallische oder keramische Biomaterialien aufgebracht wurden, sind jedoch weitestgehend erfolglos verlaufen.

So beschreibt die WO 92/00047 ein Herstellungsverfahren für spezielle, das Zellwachstum beeinflussende Implantate, bei denen jedoch das Mediatormolekül nicht kovalent gebunden ist. Darüber hinaus wird in der WO 90/09798 ein Verfahren beschrieben, bei dem die Immobilisierung eines Interleukins mittels Glutaraldehyd auf einem Polymer beschrieben wird, allerdings wird in dieser Druckschrift bei der Verwendung von Metallen und Metalloxyden kein Aktivierungsschritt erwähnt. Darüber hinaus wird in der US-A-5,330,911 die chemische Modifizierung einer Oberfläche und die kovalente Anlagerung kleiner Peptide daran zur Förderung der Zelladhäsion, insbesondere Proteine mit einer minimalen Zellrezeptor-Erkennungs-Aminosäuresequenz, die die Zelladhäsion fördert und einer Reihe von Zelladhäsionsmolekülen gemeinsam ist, beschrieben.

Die Aufgabe der vorliegenden Erfindung besteht darin, die verbesserten Biomaterialien zur Verwendung als Implantate bereitzustellen.

Erfindungsgemäß wird diese Aufgabe dadurch gelöst, dass ein Verfahren zur Immobilisierung von Mediatormolekülen auf metallischen und keramischen Materialien bereitgestellt wird. Bei dem erfindungsgemäßen Verfahren wird in einem ersten Schritt eine chemische Verbindung an die Oberfläche des Implantatmaterials als Ankermoleküle kovalent gebunden, wobei diese chemische Verbindung eine funktionelle Gruppe besitzt, die entweder selbst als Spacer- oder an der eine weitere als Spacermoleküle dienende chemische Verbindung kovalent gebunden werden kann. In einem zweiten Schritt kann ein Mediatormolekül wie ein Knochenwachstumsfaktor über funktionelle Gruppen, beispielsweise freie Aminogruppen oder Carboxylatgruppen mittels kovalenter Bindung auf dem Implantatmaterial immobilisiert werden. Dadurch wird ermöglicht, eine chemotaktische und/oder biologisch aktive Implantatoberfläche (sogenannte Juxtakrine Oberfläche) auszubilden, die zur Ansiedlung, Proliferation und Ausdifferenzierung von Knochenzellen führt.

Das erfindungsgemäße Verfahren zur Immobilisierung der Mediatormoleküle zeichnet sich dadurch aus, dass das eingesetzte Implantatmaterial aus metallischen Materialien wie Reintitan oder metallischen Titanlegierungen wie Chrom/Nickel/Aluminium/Vanadium/Kobalt-Legierungen (z.B. TiAlV4, TiAlFe2,5), Edelstähle (z.B. V2A, V4A, Chrom-Nickel 316L) oder keramischen Materialien wie Hydroxylapatit, Aluminiumoxid oder aus einer Kombination, bei der z.B. metallisches mit keramischem Material beschichtet ist, besteht. Es sind auch Kunststoffpolymermaterialien zur Verwendung als Implantatmaterial geeignet.

Es ist auch Gegenstand der Erfindung, durch Beschichtung einer koronaren Gefäßstütze (sogenannter Koronarer Stent, Länge ca. 10 mm) mit Hilfe eines Biomoleküls oder eines Mediators, z.B. BMP-2, die Spätkomplikation Restenose, die durch eine Proliferation von glatten Gefäßmuskelzellen hervorgerufen wird, therapeutisch zu verhindern oder zu mildern, um damit die Einheilung und Verträglichkeit zu fördern.

Erfindungsgemäß können die Mediatormoleküle Biomoleküle sein, die vorteilhaft für die Biokompatibilität des Implantates sind, indem sie einer möglichen Abstoßung des Implantates entgegenwirken und/oder das Einwachsen des Implantates fördern.

Als Mediatormoleküle können im vorliegenden Verfahren bevorzugt das Knochenwachstum fördernde Proteine aus der Klasse der Knochenwachstumsfaktoren "Bone Morphogenic Proteins" verwendet werden oder auch Ubiquitin. Vorteilhaft kann zur Immobilisierung ein Protein dieser Klasse allein, in Kombination mit weiteren Mitgliedern dieser Klasse oder auch zusammen mit Biomolekülen wie Proteinen anderer Klassen oder niedermolekularen Hormonen oder auch Antibiotika zur Verbesserung der Immunabwehr eingesetzt werden. Dabei können diese Moleküle auch über im biologischen Milieu spaltbare Bindungen auf der Oberfläche Immobilisiert werden.

Erfindungsgemäß wird die Oberfläche des Implantatmaterials chemisch aktiviert, wobei die Aktivierung über ein Silanderivat wie beispielsweise γ-Aminopropyltriethoxysilan oder ein Trimethylmethoxy- bzw. Trimethylchlorsilanderivat oder 3-Glycidoxypropyltrimethoxysilan erfolgt und die Reaktion sowohl in einem wäßrigen als auch in einem organischen Lösungsmittel durchgeführt wird. An die so aktivierte Oberfläche kann in einem zweiten Schritt ein als Abstandshalter fungierendes Spacermolekül kovalent gekoppelt werden. Als Spacer kann beispielsweise ein Dialdehyd wie Glutardialdehyd, ein Isothiocyanat-, oder ein Triazinderivat dienen. Weiterhin kann als Spacermolekül eine Dicarbonsäure oder ein entsprechendes Derivat wie Bernsteinsäureanhydrid verwendet werden. Nach eventueller Aktivierung der im Spacermolekül vorhandenen Kupplungsgruppe, beispielsweise einer Carbonylfunktion, mittels eines der dafür gebräuchlichen Verfahren wird das Knochenwachstum fördernde Protein über auf seiner Oberfläche zugängliche Aminogruppen kovalent mit dem Implantatmaterial verknüpft.

Erfindungsgemäß ist es ebenfalls möglich, als Spacermolekül ein Arylamin einzusetzen, was beispielsweise durch Umsetzung des durch eine Silanverbindung aktivierten Implantatmaterials mit einem durch Nitrogruppen substituierten Benzoesäurechlorids wie p-Nitrobenzoylchlorid und anschließender Reduktion der Nitrogruppe erhalten wird. In diesem Fall erfolgt die kovalente Verknüpfung des Mediatorproteins über freie Carboxylgruppen, die für diesen Zweck nach Standardverfahren aktiviert werden können.

Weiterhin umfaßt das vorliegende Verfahren die Kopplung des Mediatormoleküls nur über Ankermoleküle ohne vorherige, wie oben beispielhaft beschriebene Aktivierung der Implantatoberfläche durch Silane, wobei zu diesem Zweck z.B. Cyanbromid eingesetzt werden kann. In diesem Fall kann die kovalente Immobilisierung des Mediatormoleküls über freie Aminogruppen des Proteins erfolgen.

Das erfindungsgemäße Verfahren umfaßt die Kopplung eines Knochenwachstumsfaktors an die Oberfläche des Implantates über Spacermoleküle, deren kovalente Bindungen unter physiologischen Bedingungen nicht gespalten werden. Als vorteilhafte Weiterbildung ist ein Knochenwachstumsfaktor über Spacermoleküle, deren kovalente Bindungen für eine limitierte Freisetzung des Mediatorproteins unter physiologischen Bedingungen spaltbar sind, an die Oberfläche des Implantates gekoppelt. Alternativ ist es auch möglich, die Knochenwachstumsfaktoren ohne Zuhilfenahme eines Spacermoleküls, beispielsweise mittels der Carbodiimid-Methode, an die aktivierte Oberfläche des Implantates zu koppeln.

Gemäß einer anderen Weiterbildung des Verfahrens werden zwei oder mehrere Spacermoleküle für die Immobilisierung zumindest eines Knochenwachstumsfaktors eingesetzt.

Die Beladungsdichte eines durch das erfindungsgemäße Verfahren auf dem Implantatmaterial immobilisierten Mediatorproteins beträgt im allgemeinen 0,03 bis 2,6 µg/cm² (z.B. 1-100 pmol/cm² BMP-2). In diesem Beladungsbereich kann eine multivalente Wechselwirkung zwischen einer Zelle (z.B. 10 µm Durchmesser) und den BMP-Molekülen auf einer biologisierten Oberfläche erzielt werden, da sich ca. 10⁶-10⁸ immobilisierte Proteinmoleküle in der Adhäsionsstelle befinden.

Die Erfinder haben umfangreiche Versuche zur Klärung des Mechanismus der Anlagerung der Proteinmoleküle an die Oberfläche durchgeführt. Sie haben dabei herausgefunden, dass bei metallischen Oberflächen wie zum Beispiel bei Titan die Anlagerung über kovalente Bindungen über auf der Metalloberfläche ausgebildeten Titandioxid-Molekülen erfolgt, die bevorzugt über eine Behandlung mit verdünnter Salpetersäure in Hydroxyl-Gruppen überführt werden.

Im Gegensatz zu den im Stand der Technik bekannten Verfahren, bei denen Biomoleküle z.B auf Polymeroberflächen oder anorganischen Knochenmaterialien abgeschieden werden und lediglich über affine Wechselwirkungen mit den Polymermolekülen auf der Oberfläche des Substrates verbleiben, ist es den Erfindern hier gelungen, die Biomoleküle kovalent auf der Oberfläche zu verankern und so länger auf der Oberfläche des Implantates bereitzustellen.

Weitergehende Untersuchungen des Erfinders haben gezeigt, dass die Verankerung der Mediatormoleküle auf der Oberfläche qualitativ und quantitativ verbessert werden kann, indem man die Anzahl der auf der Oberfläche verfügbaren Metalloxid-Einheiten erhöht. Seitens des Erfinders wurde gefunden, dass die Anzahl der Oxidgruppen überraschenderweise dadurch erhöht werden kann, dass die Oberfläche des Metalles mit heißer, vorzugsweise bodensatzfreier, Chromschwefelsäure behandelt wird. Im Gegensatz zu der Erwartung, dass sich das Metall unter diesen Bedingungen auflöst, wird bei Verwendung dieser Säure eine relativ gleichmäßige Oxidschicht auf der Oberfläche des Metalles erzeugt. Das Verfahren ist so schonend, daß selbst koronare Gefäßstützen, sogenannte Stents (die z.B. aus Edelstahl oder Titan gefertigt sein können) ohne Zerstörung des dünnen empfindlichen Gitterwerkes (50-150 µm Durchmesser) beschichtet werden können. Die Oxidschicht kann dabei eine Dicke von 10 µm bis zu 100 µm erreichen und relativ "glatt" ohne Vertiefungen oder Löcher aufgebaut werden. Als Metall für das Implantat kann dabei Reintitan oder Titanlegierungen (z.B. TiAlV4, TiAlFe2,5), Aluminium oder rostfreier Stahl (z.B. V2A, V4A, Chrom-Nickel 316L) eingesetzt werden. Eine handelsübliche Chromschwefelsäure mit 92 Gew.-% H₂SO₄, 1,3 Gew.-% CrO₃ und einer Dichte von 1,8 g/cm³ beispielweise von der Firma Merck erhältlich, wird bevorzugt zur Erzielung einer dünnen glatten Schicht aus Metalloxid verwendet. Dazu wird das Metallsubstrat in die Chromschwefelsäure eingelegt und über einen Zeitraum von 1 bis zu 3 Stunden bei 100 bis 250°C behandelt, vorzugsweise 30 Minuten bei 240°C danach sorgfältig mit Wasser abgespült, danach in Wasser oder einer Lösung von 1-4 mM EDTA (Ethylendiamintetraacetat), vorzugsweise 4 mM EDTA 30 min gekocht, um auf der Oberfläche verbliebene Chromionen zu entfernen, und dann getrocknet.

Wenn eine dickere Metalloxidschicht an der Metalloberfläche und/oder eine Oxidschicht mit kleinen Mikro- und Nanoporen versehen werden soll, wird die oben beschriebene Chromschwefelsäure mit Wasser auf eine Dichte von 1,5 bis 1,6 g/cm³ verdünnt. Bei einer dann folgenden wie oben beschriebenen Behandlung des Metallimplantatoberfläche mit der so verdünnten Säure wird eine "rauhe" Oberflächenschicht mit Vertiefungen und Poren ausgebildet, so dass die zur Beladung mit Mediatormolekülen zur Verfügung stehende Oberfläche vergrößert wird. Durch Einstellung unterschiedlicher Dichten an Chromschwefelsäure ist es daher möglich, eine Vielzahl verschiedener Oxidschichten unterschiedlicher Eigenschaften auf Metalloberflächen mit hoher Haftfestigkeit aufzubringen. Die Erfindung ist daher auch auf ein solches Verfahren zur Ausbildung einer thermodynamisch einheitlichen Metalloxidschicht (keine Kontaktwinkelhysterese) auf dem Implantatmaterial mittels heißer Chromschwefelsäure gerichtet.

Die Metalloxidschicht auf dem Implantatmaterial aus den oben genannten Materialien kann dann über Behandlung mit verdünnter Salpetersäure (ca. 5 Gew.-%) und anschließende Kopplung eines Silanderivates, ggfs zusätzlich eines Spacermoleküls wie oben beschrieben, aktiviert werden.

Über die Moleküle des Silanderivates oder des Spacers können dann die Mediatormoleküle über Kopplungsverfahren wie z.B. mittels Carbonyldiimidazol auf der Implantatoberfläche verankert werden.

Um die unspezifische Adsorption der Mediatormoleküle, die bis zu 30% der adsorbierten Mediatormoleküle betragen kann, auf der Metalloberfläche auszuschließen, ist es weiterhin im Rahmen der vorliegenden Erfindung bevorzugt, an die mit der Metalloxidschicht versehene Oberfläche des Implantates zunächst eine diese Adsorption verhindernde Schicht aus Spacermolekülen wie z.B. Agarose zu koppeln, an die wiederum die Mediatormoleküle gekoppelt werden. Eine Verhinderung der unspezifischen Adsorption kann sinnvoll sein, um beispielsweise einer Blockade von BMP-Rezeptoren infolge von Konformations-änderungen der BMP-Proteine nach unspezifischer Adsorption an der Oberfläche vorzubeugen. Die Erfindung ist daher auch auf ein solches Verfahren zur Ausbildung einer die unspezifische Bindung verhindernden Beschichtung auf der Metalloxidschicht und anschließender Kopplung der Mediatormoleküle gerichtet. Die Verwendung einer Beschichtung aus Agarose zu diesem Zweck ist dabei bevorzugt.

Als Implantatmaterial kann auch ein keramisches Material wie beispielsweise Hydroxylapatit verwendet werden. Das Hydroxylapatit sollte dabei zunächst durch Behandlung mit Aminoalkylsilan aktiviert werden und dann mit einem Kopplungsmittel wie Carbodiimidazol umgesetzt werden. Im nächsten Schritt kann eine Kopplung der Mediatormoleküle wie BMP oder Ubiquitin an die Oberfläche erfolgen. Die Verwendung von Spacermoleküle ist bei Einsatz von Hydroxylapatit nicht unbedingt erforderlich.

Falls unter den Kopplungsbedingungen die eingesetzten Mediatoren im Medium schwerlöslich sind, kann die Löslichkeit durch Zugabe von Tensiden/Detergentien erhöht und die Reaktion durchgeführt werden. So können bei ph-Werten > 6 schwerlösliche Knochenwachstumsfaktoren und andere Mediatoren durch ionische oder nichtionische Detergentien im Konzentrationsbereich 0.05-10%, vorzugsweise 1 -5 Gew.%, insbesondere bei 0.066% SDS bei ph-Werten > 6, insbesondere bei ph 8-10 für kovalente Kopplungsverfahren im alkalischen pH-Bereich ohne Verlust der biologischen Aktivität in Lösung gehalten werden.

Der Einfluß der nach dem erfindungsgemäßen Verfahren modifizierten Materialien auf Knochenzellen oder Osteoblastenzellinien (MC3T3-E1) wurde in Zellkultursystemen untersucht, wobei die modifizierten Materialien zu diesem Zweck in Plättchenform vorgelegt wurden. Dabei wurde beobachtet, dass es nach dem Aufbringen von Zellen zur Ausbildung konfluenter Zellrasen und zu Funktionsänderungen durch BMP-2 (z.B. Synthese von alkalischer Phosphatase) auf den Materialien kam.

Anhand der folgenden Beispiele wird die vorliegende Erfindung weiter veranschaulicht. Die Versuche wurden mit eigens gentechnologisch hergestelltem oder kommerziellen (Fa. Biochrom KG/Seromed, Berlin) hochreinem humanem BMP2 sowie Ubiquitin durchgeführt.

### Beispiel 1: Immobilisierung von BMP auf pulverförmigem Titan mit Spacer

### a) Herstellung einer reaktionsfähigen Implantatoberfläche

0,5 g Titanpulver (Partikeldurchmesser 50-100 µm) werden zu 9 ml destilliertem Wasser und je nach Substitutionsgrad 0.2-2.0 ml 10% (v/v) γ-Aminopropyltriethylethoxysilan gegeben und unter Rühren wird der pH des Reaktionsansatzes durch Zugabe von 6 N HCl auf einen Wert zwischen 3 und 4 eingestellt. Nach erfolgter pH Regulierung wird die Reaktionslösung in einem Wasserbad für 2 h bei 75° C inkubiert. Anschließend wird das aktivierte Metall mittels einer Nutsche abgetrennt, mit ca. 10 ml dest. Wasser gewaschen und in einem Trockenschrank bei 115° C getrocknet.

### b) Aktivierung der Implantatoberfläche und Einfügung eines Spacermoleküls

0,5 g des mit dem Aminoalkylsilan derivatisierten Metallpulvers wird zu 12,5 ml 2,5 %igem Glutaraldehyd in 50 mM NaH₂PO₄, pH 7,0 gegeben. Die Reaktion wird solange durchgeführt, bis zur Umsetzung bzw. bis eine Farbänderung beobachtet wird. Anschließend wird das Reaktionsprodukt über einen Filter abgetrennt und ausgiebig mit dest. Wasser gewaschen.

### c) Immobilisierung des Proteins

Zu dem gewaschenen Reaktionsprodukt mit Glutaraldehyd wird BMP in einer Menge von 0.1-3.0 mg/g Titanpulver, 0,066% Natriumdodecylsulfat (SDS) bei neutralem pH hinzugegeben und über Nacht bei 4 °C inkubiert.

### Beispiel 2: Immobilisierung von BMP auf pulverförmigem Titan ohne Spacer

### a) Herstellung einer reaktionsfähigen Implantatoberfläche

Die Herstellung einer reaktionsfähigen Implantatoberfläche erfolgte auf gleiche Weise wie in Beispiel 1.

### b) Aktivierung der Implantatoberfläche

1.0 g des mit dem Aminoalkylsilan-Derivat derivatisierten Metalls wird zu 50 ml 0.03M H₃PO₄ mit einem eingestellten pH 4.0 gegeben. Dazu wird 100-200 mg eines wasserlöslichen Carbodiimids, beispielsweise 1-Cyclohexyl-3-(2-Morpholinoethyl) carbodiimid-methoxy-p-toluolsulfonat, gegeben.

### c) Immobilisierung des Proteins

Zu dem o.g. aktivierten Titanpulver wird BMP direkt in einer Menge 0.1-3.0 mg/g Titanpulver hinzugegeben und über Nacht bei 4 °C inkubiert.

### Beispiel 3: Immobilisierung von BMP auf plättchenförmigem Titan mit Spacer

### a) Herstellung einer reaktionsfähigen Implantatoberfläche

Die Aktivierung der Implantatoberfläche erfolgte auf gleiche Weise wie in Beispiel 1. Anstelle von Titanpulver wurde lediglich die gleiche Menge Titanplättchen eingesetzt.

### b) Aktivierung der Implantatoberfläche und Einfügung eines Spacermoleküls

Das mit dem Aminoalkylsilan-Derivat aktivierte Metallplättchen wird zu 12,5 ml 2,5 %igem Glutaraldehyd in 50 mM NaH₂PO₄, pH 7,0 gegeben. Die Reaktion wird solange durchgeführt, bis eine Farbänderung beobachtet wird. Anschließend wird das Reaktionsprodukt über einen Filter abgetrennt und ausgiebig mit dest. Wasser gewaschen.

### c) Immobilisierung des Proteins

Zu dem gewaschenen Reaktionsprodukt mit Glutaraldehyd wird BMP in einer Menge 0.1-3.0 mg/g Titanplättchen bei neutralem pH hinzugegeben und über Nacht bei 4 °C inkubiert.

### Beispiel 4: Immobilisierung von BMP auf plättchenförmigem Titan ohne Spacer

### a) Herstellung einer reaktionsfähigen Implantatoberfläche

Die Aktivierung der Implantatoberfläche erfolgte auf gleiche Weise wie in Beispiel 1. Anstelle von Titanpulver wurde lediglich die gleiche Menge Titanplättchen eingesetzt.

### b) Aktivierung der Implantatoberfläche

Das mit dem Aminoalkylsilan derivatisierte Metallplättchen wird zu 50 ml 0.03 M H₃PO₄ mit einem eingestellten pH 4.0 gegeben. Dazu werden 100-200 mg eines wasserlöslichen Carbodiimids beispielsweise 1-Cyclohexyl-3-(2-Morpholinoethlyl)-carbodiimid-methoxy-p-toluolsulfonat gegeben.

### c) Immobilisierung des Proteins

Zu dem o.g. Kopplungsansatz wird BMP direkt in einer Menge 0.3-3.0 mg/g Titanplättchen hinzugegeben und bei pH 4.0 über Nacht bei 4 °C inkubiert.

### Beispiel 5: Slow-Release-Immobilisierung von BMP auf plättchenförmigem Titan ohne Spacer

### a) Aktivierung der Implantatoberfläche

1 Titanplättchen (0.5 x 1.0 cm) in einer Dicke von 0.1 bis 0.5 mm wird in 25 ml aqua dest. gegeben. Der pH wird auf 10-11 eingestellt und 1 g CNBr wird unter Konstanterhaltung des pH-Wertes bei 10-11 und unter Konstanterhaltung der Temperatur bei 15-20 °C dazugegeben. Wenn der pH-Wert konstant bleibt, ist die Reaktion abgeschlossen und das Metallplättchen wird mit 100 ml H₂O gewaschen.

### b) Immobilisierung des Proteins

Zu dem CNBr-aktivierten Metallplättchen wird BMP in einer Menge von 0.1-3.0 mg/g Plättchen in 0,066% SDS hinzugegeben und bei pH 9.0 über Nacht bei 4 °C inkubiert. Die Kopplungsreaktion kann auch bei pH 7.0 durchgeführt werden. Nach der Kopplung wird das Plättchen ausgiebig gewaschen. Die kovalente Bindung zwischen dem Metallplättchen und dem BMP hydrolysiert mit einer Halbwertzeit von ca. 1-4 Wochen, so dass lösliches BMP freigesetzt wird.

### Beispiel 6: Immobilisierung von BMP auf plättchenförmigen Hydroxylapatit ohne Spacer

### a) Herstellung einer reaktionsfähigen Implantatoberfläche

Man läßt Hydroxylapatit über Nacht in einer 10%igen Lösung von -Aminopropyltriethylethoxysilan in Toluol unter Refluxbedingungen reagieren. Danach wird das Hydroxylapatit mit Toluol gewaschen und getrocknet.

### b) Aktivierung der Implantatoberfläche

1.0 g des mit dem Aminoalkylsilan-Derivat reaktionsfähig gemachten Apatits wird zu 50 ml 0.03M H₃PO₄ mit einem eingestellten pH 4.0 gegeben. Dazu wird 100-200 mg eines wasserlöslichen Carbodiimids beispielsweise 1-Cyclohexyl-3-(2-Morpholinoethlyl) -carbodiimid-methoxy-p-toluolsulfonat gegeben.

### c) Immobilisierung des Proteins

Zu dem o.g. Kopplungsansatz wird BMP direkt in einer Menge von 1-10 mg/g Hydroxylapatit hinzugegeben und bei pH 4.0 über Nacht bei 4 °C inkubiert.

### Beispiel 7: Immobilisierung von BMP auf plättchenförmigen Hydroxylapatit mit Spacer

### a) Herstellung einer reaktionsfähigen Implantatoberfläche

Man läßt Hydroxylalapatit über Nacht in einer 10%igen Lösung von g-Aminopropyltriethylethoxysilan in trockenem Toluol unter Refluxbedingungen reagieren. Danach wird das Hydroxylapatit mit Toluol gewaschen und getrocknet.

### b) Aktivierung der Implantatoberfläche und Einfügung eines Spacermoleküls

0,5 g des mit dem Aminoalkylsilan-Derivat reaktionsfähig gemachten Apatits wird zu 12,5 ml 2,5 %igem Glutaraldehyd in 50 mM NaH₂PO₄, pH 7,0 gegeben. Die Reaktion wird solange durchgeführt, bis zur Umsetzung bzw. bis eine Farbänderung beobachtet wird. Anschließend wird das Reaktionsprodukt über einen Filter abgetrennt und ausgiebig mit dest. Wasser gewaschen.

### c) Immobilisierung des Proteins

Zu dem o.g. Kopplungsansatz wird BMP direkt in einer Menge von 1-10 mg/g Hydroxylapatit hinzugegeben und bei pH 7.0 über Nacht bei 4 °C inkubiert.

Anstelle der in den Herstellungsbeispielen unter 2a, 4a und 6a angegebenen Methoden kann eine reaktionsfähige Implantatoberfläche auch auf folgende Weise bereitgestellt werden. Dazu läßt man 0.5g Metallpulver, 1 Metallplättchen oder 1g Apatit über Nacht in einer 2%igen Lösung von 3-Glycidoxypropyltrimethoxysilan (GPS) in trockenem Toluol unter Refluxbedingungen reagieren. Danach wird das jeweilige Probenmaterial mit Toluol gewaschen und im Vakuum getrocknet. Zur Bildung eines reaktionsfähigen primären Hydroxyderivates aus dem Epoxyderivat werden zu den o.g. Mengen der GPS-Derivate 15 ml Essigsäure/H₂O (90:10), die 0.83 g Natriumperiodat enthält, gegeben. Der Ansatz wird 2 Stunden bei Raumtemperatur durchmischt und inkubiert. Dann wird die flüssige Phase entfernt und mit Wasser, Azeton und Diethylether gewaschen (jeweils 20 ml). Es kann dann eine der o.g. Aktivierungsreaktionen angeschlossen werden.

Anstelle der in den Herstellungsbeispielen unter 2b, 4b und 6b angegebenen Methoden kann die Aktivierung der Implantatoberfläche auch auf folgende Weise erfolgen. Hierzu werden 0.5 g des mit dem Aminoalkylsilan derivatisierten Metallpulvers (2a) oder ein mit Aminoalkylsilan derivatisiertes Metallplättchen (4a) oder 1.0 g des mit dem Aminoalkylsilan-Derivat reaktionsfähig gemachten Apatits (6a) werden mit 50 ml wasserfreiem Azeton (<0.3%) gewaschen. Dann werden 10 ml einer Lösung 3%igen Carbonyldiimidazol/Azetonlösung zu dem silanderivatisierten Material hinzugegeben und 30 Minuten bei Raumtemperatur inkubiert. Danach wird wiederum mit 20 ml Azeton gewaschen, und es kann dann die Kupplung mit dem Protein BMP erfolgen.

### Beispiel 8: Überprüfung der biologischen Aktivität von immobilisierten BMP in Zellkultur nach Bingmann

In diesem Test wird die biologische Wirksamkeit von BMP in vitro an Primärkulturen aus Knochenexplantaten (Meerschweinchen Kalvarienzellen) untersucht: Adhäsionszahl, Anwachsen, Proliferation, Funktionsänderungen in der Hormonstimulierbarkeit und der Ausbreitung reizinduzierter ionaler Signale (z.B. Calcium- und H⁺-Ionen). Die Metallprobenkörper (Plättchen) werden so mit BMP beschichtet, dass eine Hälfte des Plättchens biologisiert ist und die andere Hälfte als Kontrolle nicht. Erste Ergebnisse belegen, dass die mit BMP-beschichteten Plättchen eine deutliche Punktionsänderung der Knochenzellen bewirken.

### Beispiel 9. Beschichtung von Titanpulver mit Protein

### a) Hydroxylierung mit Salpetersäure

2g Titanpulver (verdüst <60 µm) wird 2h bei 80°C in 5% HNO3 unter Rückfluß gerührt. Danach wird das Pulver abgefrittet und mit 500 ml Wasser gewaschen (pH = 6-7). Das Pulver wird weiter mit 30 ml Ethanol trocken gewaschen.

### b) Silanisierung mit 3-Aminopropyltriethoxysilan (APS)

1g hydroxyliertes Titanpulver wird in 45 ml trockenem Toluol suspendiert und mit 5ml APS versetzt unter Schutzgas Stickstoff (arbeiten im Atmosbag). Die Suspension wird 4h unter Rückfluß gekocht. Danach wird abgefrittet und mit 200 ml Toluol und 100 ml Ethanol gewaschen. Die Substanz wird mit Aceton getrocknet

### c) Aktivierung des Silanpulvers mit Carbonyldiimidazol (CDI)

750 mg CDI werden in 15 ml trockenem Aceton gelöst und mit 300 mg des Produktes aus b) versetzt. Die Mischung wird 3h bei RT gerührt und dann abgefrittet. Im weiteren wird mit 50ml Aceton und 50 ml Wasser gewaschen.

### d1) Kopplung mit 125I-Ubiquitin

Ubiquitin wurde mit Hilfe von Chloramin T nach einer bekannten Methode 125-Iodiert. 100mg des Silanpulvers aus c) werden in 1 ml einer Pufferlösung aus 50 mM NaPhosphatpuffer, pH 10.0 suspendiert, in dem 1 mg/ml 125I-Ubiquitin einer (spez. Radioaktivität von 5000-20000 cpm/µg) gelöst ist. Die Ubiquitinkonzentration kann zwischen 0.01 und 1.0 mg/ml liegen. Das Gemisch wird am Rad 2h bei RT und dann über Nacht gerührt. Der Überstand wird abpipettiert. Danach wird 3mal mit 1 ml Puffer gewaschen. Dann wird 4mal mit einer Lösung 0.1 M NaOH, 1% Natriumdodecylsulfat (SDS) gewaschen und noch zwei weitere Male mit Puffer und 2 Mal mit Wasser gewaschen. Das mit 125I-Ubiquitin beschichtete Titanpulver wird in einem kleinen Eppendorfgefäß mit 1 ml Aceton vermischt. Der Uberstand wird abpipettiert und das Pulver über Nacht im Ölpumpenvakuum getrocknet.

Kontrollen werden unter den gleichen Bedingungen mit dem aktivierten und/oder nicht aktivierten Produkt aus b) durchgeführt (Siehe Tabelle 1).

### d2) .Kopplung mit 125I-BMP-2

Die Kopplung von BMP-2 erfolgt analog zum Ubiquitin mit dem Unterschied, dass als Puffer 50 mM NaBorat, 0.066% SDS bei pH 10 verwendet wird. Die Konzentration von BMP-2 lag zwischen 0.01-1 mg/ml.

**Tabelle 1.**

| Proteinkopplung an Titanpulver am Beispiele des Proteins 125I-Ubiquitin | |
|---|---|
| | Immobilisiertes 125I-Ubiquitin auf Titanpulver (2400 cm2/g) |
| | µg/cm2 |
| A. Adsorption: | |
| Ubiquitin auf reinem Titanpulver | 0.638 |
| Ubiquitin auf APS-modifiziertem Titanpulver | 0.417 |
| nach NaOH/SDS Beh. | 0.107 |

| B.Kovalente Kopplung von Ubiquitin* | |
|---|---|
| Experiment Methode 1: | |
| Kontrolle | 0.107 |
| kovalente Kopplung | 0.122 |

| Experiment Methode 2: | |
|---|---|
| Kontrolle | 0.035 |
| kovalente Kopplung | 0.094 |

| | |
|---|---|
| *Definition des kovalent gebundenen Proteins: Die Menge Protein, die nach Waschen mit 0.1 M NaOH/1%SDS gemessen wird (siehe Methode). | |

### Beispiel 10 Beschichtung von Titanplättchen mit einer Oxidschicht zur Erhöhung der Proteinbindungskapazität

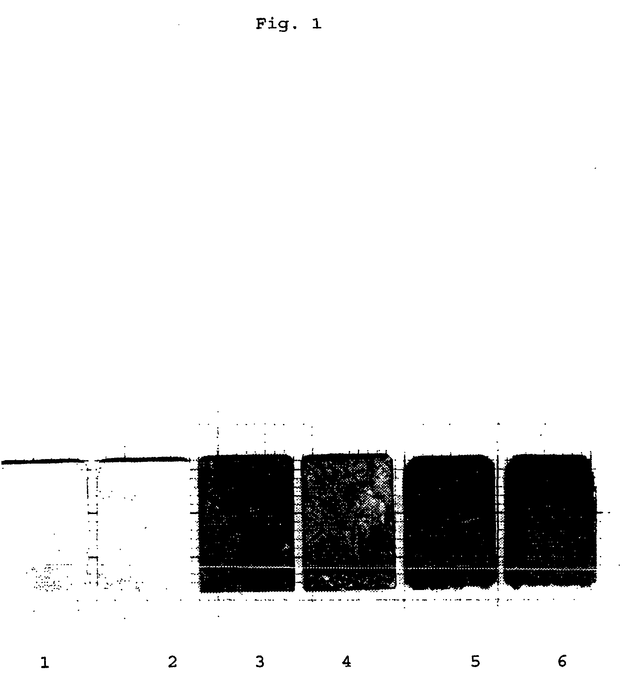

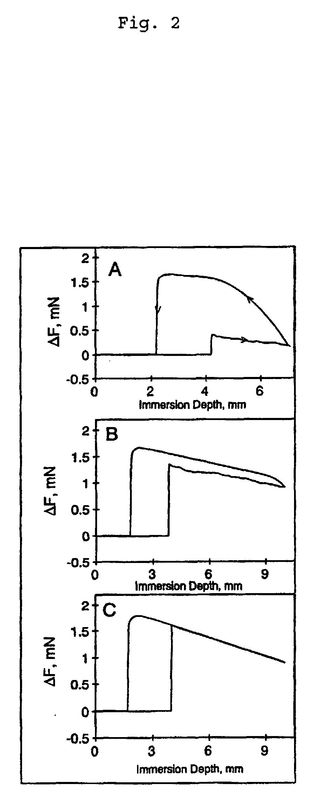

Die Oxidierung der Titanplättchen (jeweils ca. 0,5 x 1cm) wird in kochender Chromschwefelsäure bei einer Temperatur von 190-200°C für 1.5 h durchgeführt. Die durch die Oxidation grau gewordenen Plättchen werden gründlich mit Wasser abgespült. Danach werden die Plättchen 30 min in Wasser gekocht. Die Plättchen werden bei Raumtemperatur (RT) an der Luft getrocknet (Siehe Fig. 1 und 2 - Bei den in Figur 1 gezeigten Plättchen sind die Plättchen 1 und 2 unbehandelt, die Plättchen 3 und 4 mit Chromschwefelsäure der Dichte 1,8 g/cm³, die Plättchen 5 und 6 mit solcher der Dichte 1,6 behandelt). Eine EDX-Analyse (Energy Dispersive Analysis of X-rays) unter rasterelektronenmikroskopischer Kontrolle der neuen Schicht ergab zu 99% TiO2.

Wie in Fig. 1 gezeigt, sind die oxidierten TiO2-Plättchen deutlich dunkler gefärbt und haben den metallischen Glanz vollständig verloren.

Die in Fig. 2 gezeigten Hysterese-Diagramme erbringen den Nachweis der erfolgreichen Oxidationsbehandlung. Der Nachweis der unterschiedlichen Oberflächen der Titanplättchen erfolgte dabei über die Wilhelmy Plate Methode. Die Meßwerte für die einzelnen Plättchen A, B und C sind wie folgt:

| | |
|---|---|
| A. Ungereinigt | θ_{Vor} = 76.2°, θ_{Rück}= 18.2°, Hysterese: groß |
| B. Gereinigt | θ_{Vor} = 36.5°, θ_{Rück}= 21.1°, Hysterese: klein |
| C. Oxydiert | θ_{Vor} = 20.0°, θ_{Rück}= 15.0°, Hysterese: keine |

Entscheidend sind der Vorrückwinkel (ΘVor) und die Hysterese. Man sieht, dass das ungereinigte Plättchen (A) mit einem Kontaktwinkel (ΘVor) von 76° sehr hydrophob ist. Die große Hysteresefläche spricht für Verunreinigungen. Die gereinigten polierten Plättchen (B) zeigen schon verbesserte Eigenschaften mit einem wesentlich kleineren Kontaktwinkel von 36.5° und einer starken Abnahme der Hysterese. Die besten Ergebnisse werden jedoch mit den oxydierten Plättchen erzielt (C), die nur noch einen Kontaktwinkel von 20° ohne sichtbare Hysterese d.h. eine thermodynamisch-einheitliche Oberfläche besitzen.

### Beispiel 11 Kovalente Proteinbeschichtung von Titanplättchen

### a) Hydroxylierung mit Salpetersäure

Zum Vergleich werden oxidierte oder nicht-oxidierte Titanplättchen 2h bei 80°C in 5% HNO3 unter Rückfluß erhitzt. Danach werden die Plättchen mit 500 ml Wasser gewaschen (pH = 6-7). Das Plättchen werden weiter mit 30 ml Ethanol trocken gewaschen.

### b) Silanisierung

Die nicht-oxydierten oder (wie oben beschrieben) oxydierten Titanplättchen werden in ausgeheizte Behälter für die Silanisierungsreaktion gestellt. Die Behälter sollten zuvor in einer trockenen Umgebung abkühlen, bevorzugt unter Stickstoff im Eksikkator. Unter Inertgas in einem Atmosbag (Stickstoff) werden 50 ml getrocknetes Toluol und 2.5 ml APS vermischt. Der Behälter wird an der Luft möglichst zügig mit den Plättchen bestückt und unter Inert-Gas in den Rundkolben mit dem APS/Toluol Gemisch gestellt. Er wird verschlossen und unter Rückfluß 3 h lang gekocht. (Kontaktthermometer 140°C) Die Plättchen werden dreimal mit 10 ml Trichlormethan, Aceton und Methanol gespült. Die Plättchen werden an der Luft getrocknet.

### c) Aktivierung mit Carbonyldiimidazol

Danach werden die Plättchen in ihrem Behälter in eine Lösung aus Aceton (getrocknet) und Carbonyldiimidazol gestellt. Die Lösung beinhaltet 50 ml Aceton und 2.5 g CDI: Unter Inertgas wird der Rundkolben verschlossen und 4 h bei RT gerührt. Danach werden die Plättchen dreimal mit 10 ml Aceton und Wasser gespült. Die Plättchen werden an der Luft getrocknet.

### d1) Proteinbeschichtung mit 125I-Ubiquitin

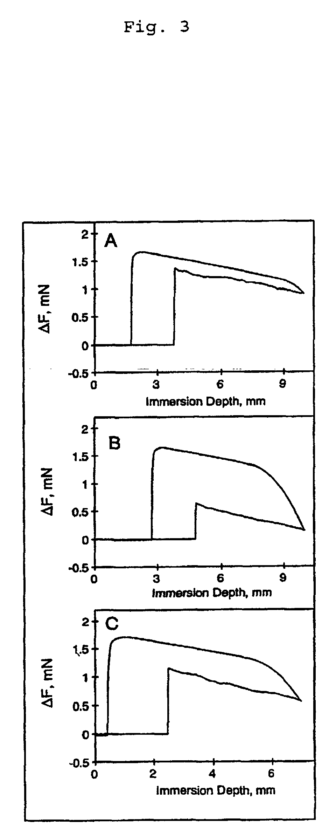

Danach werden die Plättchen einzeln in einen Pufferlösung aus 50 mM NaPhosphatpuffer pH 10 gegeben in dem eine Konzentration von 1 mg/ml 125I-Ubiquitin einer (spez. Radioaktivität von 5000-20000 cpm/µg) enthalten ist. (Die Ubiquitinkonzentration kann zwischen 0.01-1.0 mg/ml mit oder ohne 0,066% SDS liegen.) Die Plättchen werden 12-14 h bei RT geschüttelt. Danach werden die Plättchen jeweils viermal in Phosphatpuffer, einer Lösung von 0.1M NaOH, 1% Natriumdodecylsulfat (SDS) bei Zimmertemperatur gewaschen und dann 15 min bei 60° in einer Lösung von 0.1M NaOH, 1% Natriumdodecylsulfat inkubiert. Danach wird ausführlich mit Wasser gewaschen (Siehe Tabelle 2 und Fig. 3-4).

### d2) Proteinbeschichtung mit 125I-BMP-2

BMP-2 wird mit Hilfe der bekannten Bolton-Hunter Methode in einem Puffer 125 mM NaBorat, 0.066% SDS, pH 8.4 radioaktiv markiert (spez. Radioaktivität von 5000-20000 cpm/µg). Die Kopplung von 125I-BMP-2 erfolgt in einem Puffer mit 50 mM NaBorat, 0.066% SDS bei pH 10. Die Konzentration von 125I-BMP-2 kann zwischen 0.01-1.0 mg/ml liegen. Die Plättchen werden 12-14 h bei RT geschüttelt. Danach werden die Plättchen jeweils viermal in Phosphatpuffer, einer Lösung von 0.1M NaOH, 1% Natriumdodecylsulfat (SDS) bei Zimmertemperatur gewaschen und dann 15 min bei 60° in einer Lösung von 0.1M NaOH, 1% Natriumdodecylsulfat inkubiert. Danach wird ausführlich mit Wasser gewaschen.

**Tabelle 2.**

| Proteinkopplung an Titanplättchen am Beispiel des Proteins 125I-Ubiquitin | | | |
|---|---|---|---|
| | Immobilisierung von 125I-Ubiquitin | | |
| | Polierte Titanplättchen µg/cm2 | Mit Oxid beschichtete Titanplättchen µg/cm2 | |
| | | A | B |
| Adsorptions-experiment | | | |
| APS-Plättchen | 0.800 | 1.06 | 0.914 |

| Kovalentes Kopplungs-experiment | | | |
|---|---|---|---|
| "Irreversible" unspezifische Adsorption (Kontrolle) | 0.040 | 0.177 | 0.114 |
| | | 0.146 | 0.103 |
| | | | |
| App. kovalente | 0.114 | 0.500 | 0.604 |
| Kopplung | 0.106 | 0.446 | 0.589 |

**Tabelle 3.**

| Vergleichende Kopplung der Proteine 125I-Ubiquitin und 125I-BMP-2 an oxidierte Titanplättchen (mit Chromschwefelsäure Dichte 1.84 behandelt) in Gegenwart von 0.066% SDS | |
|---|---|
| | Immobilisierung von 125I-Ubiquitin Oxid-beschichtete Titanplättchen µg/cm² |
| Kovalentes Kopplungs-experiment | |
| A. "Irreversible" unspezifische Adsorption (Kontrolle) | |
| 125I-Ubiquitin (0.01 mg/ml) | 0.003 |
| 125I-BMP-2 (0.01 mg/ml) | 0.005 |
| 125I-Ubiquitin (1.0 mg/ml) | 0.172 |
| 125I-BMP-2 (1.0 mg/ml) | 0.1-0.2* |

| B. kovalente | |
|---|---|
| 125I-Ubiquitin (0.01 mg/ml) | 0.009 |
| 125I-BMP-2 (0.01 mg/ml) | 0.010 |
| 125I-Ubiquitin (1.0 mg/ml) | 0.570 |
| 125I-BMP-2 (1.0 mg/ml) | 0.4-0.6* |

| | |
|---|---|
| *geschätzt | |

Alle in Tabelle 2 dargestellten Derivate von Titanplättchen sind in der Zellkultur mit Osteoblastenabkömmlingen (MC3T3 Zellen) getestet worden. Auf allen Plättchen haben sich konfluente Zellrasen ausgebildet, die durch BMP-2 stimulierbar waren. Die oxidierten Plättchen ergaben etwa doppelt so hohe Stimulationsraten. Die Meßergebnisse lassen den Schluß zu, dass die Plättchen keine Toxizität aufwiesen, wobei die oxidierten Plättchen deutlich besser als die nicht oxidierten waren.

Fig. 3 zeigt die Kontaktwinkel- und Hystereseveränderung bei nicht oxydierten (polierten) Titanplättchen nach APS-Modifizierung und Proteinkopplung. Man kann die Beschichtung qualitativ verfolgen, doch lassen sich keine quantitativen Schlüsse ziehen. Die Meßwerte für die einzelnen Plättchen A, B und C sind wie folgt:
A. gereinigt: θ_{Vor} = 36.5°, θ_{Rück}= 21.1°, Hysterese: klein
B. APS-modifiziert: θ_{Vor} = 68.6°, θ_{Rück}= 22.6°, Hysterese: groß
C. ¹²⁵I-Ubiquitin : θ_{Vor} = 46.1°, θ_{Rück}= 17.4°, Hysterese: keine

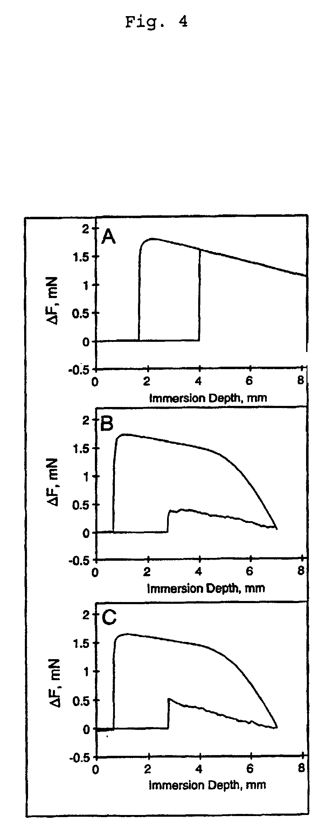

Fig. 4 zeigt Kontaktwinkel- und Hystereseveränderung bei oxydierten Titanplättchen nach APS-Modifizierung und Proteinkopplung. Man kann hier ebenfalls die Beschichtung qualitativ verfolgen, doch lassen sich keine quantitativen Schlüsse ziehen. Die Meßwerte für die einzelnen Plättchen A, B und C sind wie folgt:
A. gereinigt: θ_{Vor} = 36.5°, θ_{Rück}= 21.1°, Hysterese: klein
B. APS-modifiziert: θ_{Vor} = 76.7°, θ_{Rück}= 15.9°, Hysterese: groß
C. ¹²⁵I-Ubiquitin: θ_{Vor} = 76.9°, θ_{Rück}= 48.2°, Hysterese: groß

### Beispiel 12 Beschichtung von Titanplättchen mit Agarose zur Reduktion der unspezifischen Proteinadsorption (=Proteinabweisende Schicht)

### a) Oxidation der Agarose mit Natriumperiodat zur Dialdehyd-Agarose

Der Reaktionsansatz aus 19 g 4% Agarose-Gelkugeln (Durchmesser: 40-190 µm) z.B. Sepharose 4B, Pharmacia, 100 ml dest. Wasser, 2,5 ml 0,4 M Natriumperiodat-Lösung wurde wie folgt behandelt:

Die Agarosegelkugeln werden zuerst im Büchnertrichter mit destilliertem Wasser gewaschen und dann kurz auf der Nutsche trockengesaugt. Der feuchte Gelkuchen wird dann in 100 ml Wasser aufgenommen. Nach Zugabe von 2.5 ml 0.4 M Natriumperiodat wird die Agarosegel-Suspension 4 Stunden unter Lichtausschluß im Eisbad und danach über Nacht bei Raumtemperatur gerührt. Danach wird das Produkt mit destilliertem Wasser, 3%iger Natriumthiosulfat-Lösung und wieder mit destilliertem Wasser gewaschen und schließlich mit Aceton entwässert. Anschließend wird die fertige Agarose bei 30°C im Ölpumpen-Vakuum getrocknet. Wie die native Agarose besitzt die Dialdehyd-Agarose noch die Fähigkeit zu gelieren. Unter diesen Bedingungen werden 1 % aller Agarobiose-Einheiten oxidiert.

### b) Kopplung von Dialdehyd-Agarose an Aminopropylsilyl-Titanplättchen

### Reaktionsansatz pro Plättchen:

4 ml einer Lösung von trockener Dialdehyd-Agarose in
Kaliumphosphat-Puffer (0.1 M;
pH=7.0) bei 80 °C
Reaktionsansatz a): 0,7%ige Lösung
Reaktionsansatz b): 1,4%ige Lösung
Reaktionsansatz c): 2,1 %ige Lösung
Reaktionsansatz d): 4,0%ige Lösung

Zunächst wird die trockene Dialdehyd-Agarose bei 80°C in dem Puffer in der gewünschten Konzentration (0.7-4%) gelöst. Dann wird das Aminopropylsilyl-Titanplättchen (Herstellung siehe oben) in einer Halterung in die Lösung gegeben und zwei Stunden bei 80°C gerührt. Nach 20 Minuten werden 400 mg Natriumcyanoborhydrid zur Reduktion der gebildeten Schiff'schen Basen hinzugefügt. Das Produkt wird schließlich zur Entfernung überschüssiger Agarose mit je 15 ml 4M Natriumchlorid-Lösung und Wasser bei 80°C und schließlich mit Wasser bei Raumtemperatur gewaschen. Die Plättchen werden mit Aceton entwässert und dann bei 30°C im Vakuum über Nacht getrocknet. Die Agaroseschicht auf den Titanplättchen kann anschließend wie beschrieben mit Carbonyldiimidazol zur Kopplung von primären Aminen (z.B. an Aminosäuren oder Proteinen) aktiviert werden.

### c) Aktivierung der Agaroseschicht mit Carbonyldiimidazol

150 mg Carbonyldiimidazol werden in 3 ml Aceton gelöst und dann zu dem Agarose beschichteten Titanplättchen gegeben. Das Plättchen wird 2 Stunden bei Zimmertemperatur inkubiert und danach gründlich mit Aceton und dest. Wasser gespült.

### d1) Proteinbeschichtung mit 125I-Ubiquitin

Danach werden die Agarose-Plättchen einzeln in eine Pufferlösung aus 50 mM NaPhosphatpuffer pH 10 gegeben in dem eine Konzentration von 1 mg/ml 125I-Ubiquitin einer (spez. Radioaktivität von 5000-20000 cpm/µg) enthalten ist. (Die Ubiquitinkonzentration kann zwischen 0.01-1.0 mg/ml liegen.) Die Plättchen werden 12-14 h bei RT geschüttelt. Dann wird die Reaktion der Plättchen durch Inkubation mit 40 mg/ml Glycin in 50 mM NaPhosphatpuffer pH 10 bei Raumtemperatur für 4 Stunden abgestoppt. Danach wird jeweils mit 15 ml Wasser, 1 M NaCl und Wasser gewaschen. Bei Bedarf kann auch mit 1% SDS bei Raumtemperatur gewaschen werden.

### d2) Proteinbeschichtung mit 125I-BMP-2

BMP-2 wird mit Hilfe der bekannten Bolton-Hunter Methode in einem Puffer 125 mM NaBorat, 0.066% SDS, pH 8.4 radioaktiv markiert (spez. Radioaktivität von 5000-20000 cpm/µg). Die Kopplung von 125I-BMP-2 erfolgt in einem Puffer mit 50 mM NaBorat, 0.066% SDS bei pH 10. Die Konzentration von 125I-BMP-2 kann zwischen 0.01-1.0 mg/ml liegen. Die Plättchen werden 12-14 h bei RT geschüttelt. Dann wird die Reaktion der Plättchen durch Inkubation mit 40 mg/ml Glycin in 50 mM NaPhosphatpuffer pH 10 bei Raumtemperatur für 4 Stunden abgestoppt. Danach wird jeweils mit 15 ml Wasser, 1 M NaCl und Wasser gewaschen. Bei Bedarf kann auch mit 1% SDS bei Raumtemperatur gewaschen werden.

### d3) Derivatisierung von Quarzglasplättchen:

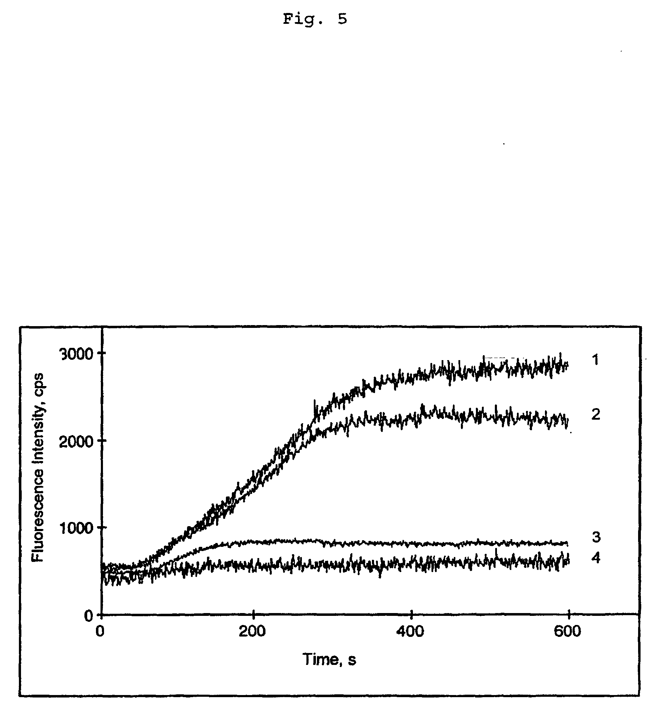

In einem analogen Verfahren können auch Quarzglasplättchen mit Agarose beschichtet werden. Auf diesen Plättchen läßt sich der proteinabweisende Effekt (Fibrinogen-Adsorption, TIRF-(Totale Innere Reflexions-Spektroskopie) Verfahren) besonders gut sichtbar machen. Fig. 5 zeigt die Reduktion der unspezifischen Adsorption von Fibrinogen durch Agarosebeschichtung von Quarzglasplättchen gemessen in Abhängigkeit von der Zeit im TIRF-Online-Verfahren. Die Adsorption von Fibrinogen (Konzentration 0.01 mg/ml) wurde in 50 mM Tris/HCl, 150 mM NaCl, 0.1 mM EDTA, pH 7.4 durchgeführt. Die Tryptophanfluoreszenz wurde bei 290 nm angeregt, und die Emission wurde bei 350 nm mit einem Fluoreszenzspektrophotometer (Spex Pluorolog 112XI) unter TIRF-Bedingungen gemessen. Die Agarose wurde in monomerer Form kovalent an die Aminofunktion des Aminopropylsilylrestes gebunden. cps: counted photons per second. Dabei bedeuten
Kurve 1. Aminopropylsilyl modizierte Quarzglasplatte
Kurve 2. Unmodizierte Quarzglasplatte (Kontrolle)
Kurve 3. Quarzglasplatte kovalent mit 0.7% Agarose beschichtet
Kurve 4. Quarzglasplatte kovalent mit 4% Agarose beschichtet

### Beispiel 13 Proteinbeschichtung von porösem Hydroxylapatit (einem Knochenersatzmaterial)

### a) Vorbereitung des Hydroxylapatits

Es wurden folgende Materialien verwendet:
a. Poröses Hydroxylapatit (aus Rinderknochen gewonnen) z.B. Endobon, Merck, Dichte: 1,289 g/cm3
b. 125I-Ubiquitin oder 125I-BMP-2

3 ml getrocknetes Toluol wird unter Stickstoff mit 0.15 ml Aminopropylsilan (APS) vermischt. Dann wird das poröse Hydroxylapatit (150 mg) hinzugegeben und 5 Stunden lang unter Rückfluß gekocht. Danach wird das Hydroxylapatit 3 mal mit Aceton, 3 mal mit Chloroform und 3 mal mit Methanol abgespült. Das poröse Hydroxylapatit wird dann in eine Lösung aus trockenem Aceton (3ml) und 150 mg Carbonyldiimidazol unter Stickstoff abgefüllt und 3 Stunden lang bei RT gerührt. Danach wird 3 mal mit 10 ml Aceton gespült.

### b1) Kopplung von Proteinen mit 125I-Ubiquitin

Das Hydroxylapatit aus a) wird in 1 ml Phosphatpuffer (50 mM) pH 10 überführt. In diesen Phosphatpuffer wird 20 µl Ubiquitin (ca: 50 mg/ml) gegeben und 10 µl radioaktives Ubiquitin ( spez. Radioaktivität der Endlösung: 32600 cps/µg) hinzugesetzt. Die Lösung wird vermischt und zunächst 2 Stunden lang am Rad bei RT gerührt. Danach wird weitere 24 h bei 4°C gerührt. Danach wird das modifizierte Hydroxylapatit mit Wasser 3mal abgespült, dann 4 mal mit einer Lösung 0.1 M NaOH, 1% Natriumdodecylsulfat (SDS) und dann 3mal mit Wasser. Die Radioaktivität wird im Gammacounter gemessen und der Substitutionsgrad bestimmt. Kontrollen werden mit gewaschenem Hydroxylapatit und/oder mit APS beschichtetem Hydroxylapatit durchgeführt (siehe Tabelle 4).

### b2) Kopplung von Proteinen mit 125I-BMP-2

Das Hydroxylapatit aus a) wird in 1 ml 50 mM Na-Boratpuffer, 0.066% SDS, pH 10 überführt. Die Kopplung von 125I-BMP-2 (spez. Radioaktivität s.oben) erfolgt im gleichen Puffer (50 mM NaBorat, 0.066% SDS bei pH 10) bei Inkubation für 2 Stunden bei Raumtemperatur. Dann wird weitere 24 Std. bei 4 °C gerührt. Danach wird das modifizierte Hydroxylapatit mit Wasser 3mal abgespült, dann 4 mal mit einer Lösung 0.1 M NaOH, 1% Natriumdodecylsulfat (SDS) und dann 3mal mit Wasser. Die Radioaktivität wird im Gammacounter gemessen und der Substitutionsgrad bestimmt. Kontrollen werden mit gewaschenem Hydroxylapatit und/oder mit APS beschichtetem Hydroxylapatit durchgeführt. Bei der Kopplung kann die Konzentration von 125I-BMP-2 zwischen 0.01-1.0 mg/ml liegen.

**Tabelle 4.**

| Kopplung von Protein an porösem Hydroxylapatit am Beispiel des Proteins 125I-Ubiquitin. | |
|---|---|
| | Poröses Hydroxylapatit Gekoppeltes Ubiquitin µg/g |
| Kontrolle | 6 |
| 125I-Ubiquitin | 24 |
| | 30 |

## Patentansprüche

1. Verfahren zur Immobilisierung von Mediatormolekülen auf Implantatmaterialen, **dadurch gekennzeichnet, dass** in einem ersten Schritt Ankermoleküle an die chemisch aktivierte Oberfläche des Implantatmaterials kovalent gebunden werden, wobei diese Ankermoleküle funktionelle Gruppen besitzen, an der weitere chemische Verbindungen kovalent gebunden werden können, und in einem zweiten Schritt Mediatormoleküle über diese funktionellen Gruppen auf dem Implantatmaterial immobilisiert werden, wobei das Implantatmaterial aus einem Material aus der Gruppe der Metalle, der metallischen Legierungen, der keramischen Materialien oder Kombinationen davon ausgewählt wird.

2. Verfahren zur Immobilisierung von Mediatormolekülen auf Implantatmaterialen, **dadurch gekennzeichnet, dass** in einem ersten Schritt Ankermoleküle an die chemisch aktivierte Oberfläche des Implantatmaterials kovalent gebunden werden, wobei diese Ankermoleküle funktionelle Gruppen besitzen, an der weitere chemische Verbindungen kovalent gebunden werden können, und in einem zweiten Schritt Mediatormoleküle über diese funktionellen Gruppen auf dem Implantatmaterial immobilisiert werden, wobei als Mediatormoleküle Knochenwachstumsfaktoren aus der Klasse der BMP-Proteine, Ubiquitin, Antibiotika oder Mischungen davon verwendet werden.

3. Verfahren nach Anspruch 2, **dadurch gekennzeichnet, dass** das Implantatmaterial aus einem Material aus der Gruppe der Metalle, der metallischen Legierungen, der keramischen Materialien oder Kombinationen davon ausgewählt wird.

4. Verfahren nach einem der Ansprüche 1 bis 3, **dadurch gekennzeichnet, dass** in einem Zwischenschritt zwischen dem ersten und zweiten Schritt Spacermoleküle an die Ankermoleküle aus dem ersten Schritt gebunden werden, und diese Spacermoleküle weitere funktionelle Gruppen zur kovalenten Bindung weiterer Moleküle besitzen, und in dem zweiten Schritt die Mediatormoleküle über die funktionellen Gruppen der Spacermoleküle auf dem Implantatmaterial immobilisiert werden.

5. Verfahren nach Anspruch 1 oder Anspruch 2, **dadurch gekennzeichnet, dass** zumindest ein Teil der chemischen Bindungen der Mediatormoleküle an die Oberfläche des Implantatmaterials so modifiziert ist, dass die Bindungen unter physiologischen Bedingungen gespalten werden können.

6. Verfahren nach einem der vorhergehenden Ansprüche, **dadurch gekennzeichnet, dass** als Knochenwachstumsfaktor BMP-2 oder BMP-7 verwendet wird.

7. Verfahren nach einem der vorhergehenden Ansprüche, **dadurch gekennzeichnet, dass** die Oberfläche des Implantatmaterials vor der kovalenten Bindung der Ankermoleküle mit einer Oxidschicht versehen wird.

8. Verfahren nach Anspruch 7, **dadurch gekennzeichnet, dass** die Oberfläche des Implantatmaterials, ausgewählt aus Titan, Titanlegierungen, Aluminium oder rostfreiem Stahl, vor der kovalenten Bindung der Ankermoleküle durch Behandlung mit Chromschwefelsäure über einen Zeitraum von 0,5 bis zu 3 Stunden bei 100 bis 250°C mit einer Oxidschicht versehen wird.

9. Verfahren zur Aufbringung einer Oxidschicht auf metallischen Substraten, **dadurch gekennzeichnet, dass** die Oberfläche des metallischen Substrates über einen Zeitraum von 0,5 bis zu 3 Stunden bei 100 bis 250°C mit heißer Chromschwefelsäure behandelt wird.

10. Verfahren nach Anspruch 8 oder 9, **dadurch gekennzeichnet, dass** die Chromschwefelsäure eine Dichte von mehr als 1,40 g/cm³ hat.

11. Verfahren nach Anspruch 9 oder 10, **dadurch gekennzeichnet, dass** es sich bei dem metallischen Substrat um ein Implantat handelt.

12. Verfahren nach Anspruch 4, 7 oder 8, **dadurch gekennzeichnet, dass** im ersten Schritt Aminoalkylsilanmoleküle als Ankermoleküle an die Implantatoberfläche gebunden werden, im Zwischenschritt Agarosemoleküle als Spacermoleküle kovalent an die Ankermoleküle gebunden werden, und im zweiten Schritt ein Knochenwachstumsfaktor aus der Klasse der BMP-Proteine oder Ubiquitin als Mediatormoleküle an die Agarose kovalent gekoppelt werden.

13. Implantat, erhältlich nach einem der Ansprüche 1-8 oder 12.

14. Implantat nach Anspruch 13, **dadurch gekennzeichnet, dass** das Implantatmaterial aus Titan, Titanlegierungen, Aluminium, rostfreien Stahl oder Hydroxylapatit besteht.

## Claims

1. Method for immobilising mediator molecules on implant materials, **characterised in that**, in a first step, anchor molecules are covalently bonded to the chemically activated surface of the implant material, these anchor molecules having functional groups to which further chemical compounds may be covalently bonded, and, in a second step, mediator molecules are immobilised on the implant material by means of those functional groups, the implant material being selected from a material from the group of metals, metal alloys, ceramic materials or combinations thereof.

2. Method for immobilising mediator molecules on implant materials, **characterised in that**, in a first step, anchor molecules are covalently bonded to the chemically activated surface of the implant.material, these anchor molecules having functional groups to which further chemical compounds may be covalently bonded, and, in a second step, mediator molecules are immobilised on the implant material by means of those functional groups, bone growth factors from the class of the BMP proteins, ubiquitin, antibiotics or mixtures thereof being used as the mediator molecules.

3. Method according to claim 2, **characterised in that** the implant material is selected from a material from the group of metals, metal alloys, ceramic materials or combinations thereof.

4. Method according to any one of claims 1 to 3, **characterised in that**, in an intermediate step between the first and second step, spacer molecules are bonded to the anchor molecules from the first step and these spacer molecules have further functional groups for the covalent bonding of further molecules, and, in the second step, the mediator molecules are immobilised on the implant material by means of the functional groups of the spacer molecules.

5. Method according to claim 1 or claim 2, **characterised in that** at least some of the chemical bonds of the mediator molecules to the surface of the implant material are so modified that the bonds can be split under physiological conditions.

6. Method according to any one of the preceding claims, **characterised in that** BMP-2 or BMP-7 is used as the bone growth factor.

7. Method according to any one of the preceding claims, **characterised in that** the surface of the implant material is provided with an oxide layer prior to the covalent bonding of the anchor molecules.

8. Method according to claim 7, **characterised in that**, prior to the covalent bonding of the anchor molecules, the surface of the implant material, selected from titanium, titanium alloys, aluminium or stainless steel, is provided with an oxide layer by treatment with chromosulphuric acid over a period of from 0.5 to 3 hours at from 100 to 250°C.

9. Method for applying an oxide layer to metal substrates, **characterised in that** the surface of the metal substrate is treated with hot chromosulphuric acid over a period of from 0.5 to 3 hours at from 100 to 250°C.

10. Method according to claim 8 or 9, **characterised in that** the chromosulphuric acid has a density of more than 1.40 g/cm³.

11. Method according to claim 9 or 10, **characterised in that** the metal substrate is an implant.

12. Method according to claim 4, 7 or 8, **characterised in that**, in the first step, aminoalkylsilane molecules are bonded as anchor molecules to the implant surface, in the intermediate step agarose molecules are covalently bonded as spacer molecules to the anchor molecules, and, in the second step, a bone growth factor from the class of the BMP proteins or ubiquitin is covalently coupled as mediator molecules to the agarose.

13. Implant, obtainable according to any one of claims 1 to 8 or 12.

14. Implant according to claim 13, **characterised in that** the implant material is composed of titanium, titanium alloys, aluminium, stainless steel or hydroxyl apatite.

## Revendications

1. Procédé pour immobiliser des molécules médiatrices sur des matériaux d'implant, **caractérisé en ce que** l'on lie de manière covalente, dans une première étape, des molécules d'ancrage sur la surface chimiquement activée du matériau d'implant, où ces molécules d'ancrage possèdent des groupes fonctionnels sur lesquels d'autres composés chimiques peuvent être liés de manière covalente, et dans une deuxième étape, on immobilise les molécules médiatrices sur ces groupes fonctionnels sur le matériau d'implant, où le matériau d'implant est choisi parmi un matériau du groupe des métaux, des alliages métalliques, des matériaux céramiques ou de leurs combinaisons.

2. Procédé pour immobiliser des molécules médiatrices sur des matériaux d'implant, **caractérisé en ce que** l'on lie de manière covalente, dans une première étape, des molécules d'ancrage sur la surface chimiquement activée du matériau d'implant, où ces molécules d'ancrage possèdent des groupes fonctionnels sur lesquels d'autres composés chimiques peuvent être liés de manière covalente, et dans une deuxième étape, on immobilise les molécules médiatrices sur ces groupes fonctionnels sur le matériau d'implant, où on utilise comme molécule médiatrice, des facteurs de croissance des os de la classe des protéines BMP, l'ubiquitine, des antibiotiques ou leurs mélanges.

3. Procédé selon la revendication 2, **caractérisé en ce que** le matériau d'implant est choisi parmi un matériau du groupe des métaux, des alliages métalliques, des matériaux céramiques ou de leurs combinaisons.

4. Procédé selon l'une quelconque des revendications 1 à 3, **caractérisé en ce que** dans une étape intermédiaire entre la première et la deuxième étape, on lie des molécules espaceurs sur la molécule d'ancrage de la première étape, et ces molécules espaceurs possèdent d'autres groupes fonctionnels pour la liaison covalente d'une autre molécule, et dans la deuxième étape, les molécules médiatrices sont immobilisées par ces groupes fonctionnels des molécules espacers.

5. Procédé selon la revendication 1 ou 2, **caractérisé en ce qu'**au moins une partie des liaisons chimiques des molécules médiatrices sur la surface du matériau d'implant est modifiée de sorte que les liaisons peuvent être clivées dans les conditions physiologiques.

6. Procédé selon l'une quelconque des revendications précédentes, **caractérisé en ce que** l'on utilise comme facteur de croissance des os, la BMP-2 ou la BMP-7.

7. Procédé selon l'une quelconque des revendications précédentes, **caractérisé en ce que** la surface du matériau d'implant est munie, avant la liaison covalente des molécules d'ancrage, d'une couche d'oxyde.

8. Procédé selon la revendication 7, **caractérisé en ce que** la surface du matériau d'implant, choisie parmi le titane, un alliage de titane, l'aluminium ou l'acier inoxydable, est munie avant la liaison covalente avec les molécules d'ancrage, d'une couche d'oxyde par traitement avec de l'acide sulfo-chromique pendant une période de 0,5 à 3 heures, de 100 à 250°C.

9. Procédé d'application d'une couche d'oxyde sur des substrats métalliques, **caractérisé en ce que** la surface du substrat métallique est traité avec de l'acide sulfo-chromique chaud pendant une période de 0,5 à 3 heures, de 100 à 250°C.

10. Procédé selon la revendication 8 ou 9, **caractérisé en ce que** l'acide sulfo-chromique a une masse volumique supérieure à 1,40 g/cm³.

11. Procédé selon la revendication 9 ou 10, **caractérisé en ce que** le substrat métallique consiste en un implant.

12. Procédé selon la revendication 4, 7 ou 8, **caractérisé en ce que** dans la première étape, on lie des molécules d'aminoalkylsilane comme molécule d'ancrage sur la surface d'implant, dans l'étape intermédiaire on lie de manière covalente sur les molécules d'ancrage, des molécules d'agarose comme molécule espaceur, et dans la deuxième étape, on couple de manière covalente sur l'agarose, un facteur de croissance des os de la classe des protéines BMP ou l'ubiquitine comme molécule médiatrice.

13. Implant, pouvant être obtenu selon l'une quelconque des revendications 1-8 ou 12.

14. Implant selon la revendication 13, **caractérisé en ce que** le matériau d'implant est le titane, un alliage de titane, l'aluminium, l'acier inoxydable ou l'hydroxyapatite.
